# FASCICULE DE BREVET EUROPEEN

(11) **EP 3 082 464 B1**
(45) Date de publication et mention de la délivrance du brevet: **07.06.2023**
(21) Numéro de dépôt: 14805607.0
(22) Date de dépôt: 02.12.2014
(51) Int. Cl.: A23L 33/10, A61K 36/87, A61Q 19/08, A61K 8/97, A23L 33/105, A61K 8/9789, A61K 8/9794, A61P 17/00

(54) **COMPOSITION COMPRENANT DES EXTRAITS DE RAISIN TANNAT OU CANNONAU, PROCÉDÉ D'EXTRACTION ET UTILISATIONS**
ZUBEREITUNG ENTHALTEND WEINTRAUBENEXTRAKT, DIE EXTRAKTIONSMETHODE UND SEINE VERWENDUNG
COMPOSITION COMPRISING GRAPE EXTRACTS, THE EXTRACTION METHOD AND ITS USES

(30) Priorité: 02.12.2013 FR 1361930; 10.06.2014 FR 1455240
(43) Date de publication de la demande: 26.10.2016
(73) Titulaire: Maximaliste Limited, London W1T 3PS (GB)
(72) Inventeur: BARADAT, André, London W1T 3PS (GB); ROSSIGNOL-CASTERA, Anne, F-34400 Lunel (FR); LEMOINE, Noémie, F-34400 Lunel (FR)
(74) Mandataire: Regimbeau
(86) Numéro de dépôt international: PCT/EP2014/076161
(87) Numéro de publication internationale: WO 2015/082427

(56) Documents cités:
- WO-A1-2007/141026
- CN-A- 101 606 738
- FR-A1- 2 943 684
- JP-A- 2011 174 021
- RU-A- 2007 115 695
- US-A1- 2007 071 841
- L'OCCITANE: "Crushed Grape Polish", GNPD; MINTEL, octobre 2006 (2006-10), - octobre 2006 (2006-10), XP002724406, [extrait le 2006-10-01]
- GONZALEZ-NEVES-G BARREIRO-L GILG FRANCO-I FERRER-M MOUTOUNET-M CARONNEAU-A: "Anthocyanic composition of Tannat grapes from the south region of Uruguay", ANALYTICA CHIMICA ACTA, ELSEVIER, AMSTERDAM, NL , vol. 513, no. 1 18 juin 2004 (2004-06-18), pages 197-202, XP002730694, ISSN: 0003-2670, DOI: 10.1016/J.ACA.2003.11.078 Extrait de l'Internet: URL:http://ac.els-cdn.com/S000326700301574 5/1-s2.0-S0003267003015745-main.pdf?_tid=4 556e4de-6659-11e4-9f83-00000aacb362&acdnat =1415349613_b11b402f534dd6fc625d45ece26338 15 [extrait le 2004-01-17]
- TUBEROSO CARLO IGNAZIO GIOVANNI ET AL: "Antioxidant capacity and vasodilatory properties of Mediterranean food: The case ofCannonauwine, myrtle berries liqueur and strawberry-tree honey", FOOD CHEMISTRY, ELSEVIER LTD, NL, vol. 140, no. 4, 28 septembre 2012 (2012-09-28), pages 686-691, XP028546402, ISSN: 0308-8146, DOI: 10.1016/J.FOODCHEM.2012.09.071

## Description

La présente invention est relative aux extraits de raisins de cépage Tannat et/ou de cépage Cannonau, à leur procédé de préparation et l'utilisation de ces extraits pour les applications cosmétique, dermatologique, alimentaire et textile.

L'effet des différents vins rouge sur des cellules endothéliales de culture a été étudié par R. Corder et son équipe ; les résultats ont été publiés dans « Nature ». Ils ont montré que les vins du Gers ont une activité biologique et une teneur en OPC 2 à 4 fois supérieurs aux autres vins. La teneur en OPC plus élevée des vins du Gers est due à la nature du raisin, le Tannat ainsi qu'au mode d'extraction traditionnel qui permet une meilleure extraction des OPC. Cette étude permet d'expliquer l'origine du French paradoxe.

La peau vieillit lorsque ses cellules ne se régénèrent plus au rythme de leur destruction. Dès l'âge de trente ans, les tissus perdent leur élasticité et leur pouvoir de réguler la diffusion gazeuse (oxygène et gaz carbonique). 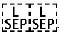 Au cours du vieillissement, le derme s'amincit et la densité des fibres augmente. Rides et ridules apparaissent et, plus tard, les stigmates tels l'aspect flasque et flétri, les petits angiomes et les comédons.

Tous ces signes témoignent de la transformation des tissus conjonctifs endo et extracellulaires et sont en relation avec les phénomènes oxydatifs qui contribuent à modifier l'apparence. Car du point de vue chimique, les principaux mécanismes du vieillissement cutané sont de nature oxydante par formation de radicaux libres.

L'oxygène intervient dans la synthèse et la dégradation des constituants de la peau - lipides, polyglucosides, vitamines, hormones, porphyrines du sang, etc. Les phénomènes d'oxydation chimique (oxydation dans laquelle intervient aussi le rayonnement lumineux) et d'oxydation enzymatique augmentent avec l'âge.

Ces mécanismes entraînent la densification du réseau des collagènes, la dégradation des élastines et la diminution du taux de glycoprotéines dans les tissus. Ils provoquent l'épaississement et le brunissement de la peau qui sont autant d'éléments de défense des structures kératinisées et mélanisées superficielles cutanées.

Au niveau de la peau, les radicaux libres s'en prennent aux membranes des cellules en dégradant le collagène, élément essentiel impliqué dans l'apparition de la ride. De plus en plus, les fabricants de produits cosmétiques introduisent dans leurs crèmes des actifs anti radicaux libres dont les polyphénols.

L'objet de ce travail est d'extraire les actifs, tels que les actifs polyphénoliques, du raisin, de la variété Tannat ou Cannonau de façon à préserver leur pouvoir antioxydant pour pouvoir bénéficier de l'effet à l'origine du French paradoxe. L'utilisation de ces extraits, issus des cépages Tannat ou Cannonau, plus particulièrement dans les applications notamment cosmétiques, dermatologiques et alimentaires, permettra de bénéficier du phénomène à l'origine du French paradoxe autrement qu'en consommant de vin du sud-ouest de la France.

L'invention porte sur la mise au point d'extraits de raisin Tannat ou Cannonau qui se présentent soit sous forme huileuse soit sous forme aqueuse, leur utilisations, en particulier leurs utilisations synergiques et leurs caractérisations.

Les actifs du raisin sont avantageusement choisis parmi les oligoéléments, les vitamines, les acides gras, les composés phénoliques du raisin, et leurs mélanges. Les oligo-éléments sont avantageusement choisis parmi le calcium, le fer, le magnésium, le phosphore, le potassium, le cuivre, le sodium, le zinc et leurs mélanges. Les vitamines sont avantageusement choisies parmi la vitamine C, la vitamine B3, la vitamine B1, la vitamine B2, la vitamine B5, la vitamine B6, la vitamine B9, le beta-carotène, la vitamine E, la vitamine K, et leurs mélanges. Les acides gras peuvent être saturés, mono-insaturés ou polyinsaturés. Les composés phénoliques sont avantageusement choisis parmi les acides phénols, les flavonoïdes, les stilbènes et leurs mélanges. Les acides phénols sont avantageusement choisis parmi les acides hydrobenzoïques, les acides hydroxycinnamiques et leurs mélanges. Les flavanoïdes sont avantageusement choisis parmi les flavanols, les proanthocyanidols, les anthocyanidols, les anthocyanosides, les flavonols, les hétérosides de flavanols, et leurs mélanges. Les flavanols et les proanthocyanidols sont avantageusement choisis parmi le catéchol, l'épicatéchol ; le gallocatéchol, l'épigallocatéchol, le procyanidol B1, le procyanidol B2, le procyanidol B3, le procyanidol B4, le procyanidol B5, le procyanidol B6, le procyanidol B7, le procyanidol B8, le procyanidol A2, et leurs mélanges. Les anthocyanidols et les anthocyanosides sont avantageusement choisis parmi le malvidol, le cyanidol, le delphinidol, le pétunidol, la paenidol, le malvidol 3-glucoside, le delphinidol 3-glucoside, le pétunidol 3-glucoside, et leurs mélanges. Les flavonols et les hétérosides de flavanols sont avantageusement choisis parmi le myrcétol, le quercétol, le myrcétol 3-glucoside, le quercétol 3-glucoside, la rutine, et leurs mélanges. Les stilbènes sont avantageusement choisis parmi le trans-resvératrol, le cis-rescératrol, le trans-picéide, le cis-picéide, et leurs mélanges.

L'invention a pour objet une composition, avantageusement cosmétique, dermatologique ou nutraceutique, comprenant
(a) un extrait huileux de raisin de cépage Cannonau et/ou de cépage Tannat,
   - Ledit extrait a un indice de péroxyde inférieur à 20 méqO₂ par kg d'extrait huileux,
   - Ledit extrait est obtenu par mélange des grains de raisins et d'au moins une huile puis extraction physique des actifs non volatils lipophiles des grains de raisin vers ladite huile par un procédé impliquant uniquement des traitements thermiques et mécaniques, sans impliquer d'autre solvant
(b) et un concentrât hydrophile de raisin de cépage Cannonau et/ou de cépage Tannat
et (c) éventuellement un excipient cosmétiquement, dermatologiquement ou nutraceutiquement acceptable.

Par la suite, l'extrait huileux de raisin de cépage Cannonau et/ou de cépage Tannat pourra être dénommé « extrait huileux » ou « extrait huileux de raisin Cannonau et/ou Tannat ».

Par l'expression « Cannonau et/ou Tannat », on entend que la matière première raisin peut être du raisin de cépage Cannonau, du raisin de cépage Tannat, ou un mélange de ces raisins. On peut aussi combiner un extrait de raisin de cépage Tannat et un extrait de raisin de cépage Cannonau.

Le procédé permettant d'extraire facilement les actifs présents dans ces raisins, on pourra utiliser tout cépage, quelque soit l'exploitation.

Les actifs du raisin comprennent les actifs mentionnés ci-dessus, et notamment des composés phénoliques ou un mélange quelconque de ces composés. Dans une variante de l'invention, ces composés sont des polyphénols, en particulier des acides phénoliques et/ou des flavonoïdes. Les flavonoïdes sont avantageusement choisis parmi la quercétine, les catéchines et leurs mélanges.

Les polyphénols constituent une famille de molécules organiques largement présente dans le règne végétal. Ils sont caractérisés par la présence de plusieurs groupements phénoliques associés en structures plus ou moins complexes généralement de haut poids moléculaire. Les polyphénols prennent une importance croissante, notamment grâce à leurs effets bénéfiques sur la santé. En effet, leur rôle d'antioxydants naturels suscite de plus en plus d'intérêt pour la prévention et le traitement du cancer, des maladies inflammatoires, cardiovasculaires et neurodégénératives. Ils sont également utilisés comme additifs pour l'industrie agroalimentaire, pharmaceutique et cosmétique.

La consommation élevée de polyphénols est associée à la réduction du taux de CHD ainsi que la réduction de l'athérosclérose dans les modèles expérimentaux. Les polyphénols du vin rouge induisent la vasodilatation endothélium dépendante. Ce type d'effet vasculaire joue très probablement un effet majeur dans l'action anti-athérosclérose.

L'extrait huileux de raisin Cannonau et/ou Tannat a avantageusement une teneur en polyphénols au moins 20 à 100 fois supérieure à la teneur en polyphénols dans l'huile naturelle non enrichie, le facteur de concentration dépendant directement des matières premières choisies et des conditions d'extraction.

L'extrait huileux a avantageusement une teneur totale en polyphénols, exprimée en équivalent épicatéchine, d'au moins 100 ppm, plus avantageusement d'au moins 200 ppm, encore plus avantageusement d'au moins 500 ppm, encore plus avantageusement de 500 ppm à 2000 ppm.

L'extrait huileux a avantageusement une teneur totale en polyphénols, exprimée en équivalent oleuropéine, d'au moins 200 ppm, plus avantageusement d'au moins 400 ppm, encore plus avantageusement d'au moins 1000 ppm, encore plus avantageusement de 1000 ppm à 4000 ppm.

On utilise en particulier les grains des raisins de cépage Cannonau ou de cépage Tannat, plus exactement les matières solides obtenues après séparation du jus.

Le tannat N est un cépage rouge de vigne. Il appartient à la famille des cotoïdes. Il est très répandu dans le sud-ouest de la France. L'identification du cépage Tannat fait appel :
- à l'extrémité du jeune rameau qui présente une forte densité de poils couchés
- aux jeunes feuilles rougeâtres à plages bronzées
- aux feuilles adultes de couleur vert foncé, de grande taille, pentagonales, entières, à trois ou cinq lobes, avec un lobe central allongé, un sinus pétiolaire peu ouvert ou fermé, des dents courtes à côtés rectilignes, une pigmentation anthocyanique des nervures moyenne, un limbe révoluté, bullé, parfois ondulé entre les nervures principales, et à la face inférieure, une densité moyenne des poils couchés
- aux baies qui sont de forme arrondie

Les 10 clones agréés de Tannat portent les numéros 398, 399, 472, 473, 474, 475, 717, 794, 944 et 1048. Un conservatoire de plus de 300 clones a été implanté en 1995 dans les Pyrénées-Atlantiques.

Le Cannonau est un cépage (grenache) très répandu en Sardaigne, produisant des vins rouges.

Les raisins de la variété Tannat ou de la variété Cannonau ont été sélectionnés pour leur richesse en polyphénols et en procyanidines.

Les raisins, matière première, sont avantageusement récoltés avant véraison, c'est-à-dire avant changement de texture et de couleur (encore appelés « raisins verts »). La croissance du grain de raisin se divise en 4 étapes : la floraison, la véraison, la maturation et la sur-maturation. La véraison se caractérise par un brusque ramollissement de la baie et changement de couleur. Il a été démontré qu'il était préférable de choisir un raisin récolté avant maturation complète du grain pour une meilleure extraction des actifs dont les polyphénols.

Avantageusement, les grains de raisin sont composés de la pellicule, des pépins et de la pulpe.

L'huile utilisée pour la préparation de l'extrait huileux est avantageusement une huile végétale, vierge, désodorisée ou raffinée, adaptée à une utilisation cosmétique, dermatologique ou nutraceutique. En particulier une huile stable, c'est-à-dire présentant une stabilité à l'oxydation compatible avec son utilisation. Si possible, l'huile doit être peu colorée et peu odorante.

L'huile choisie a de préférence une teneur significative en acides gras essentiels oméga 3 et oméga 6 afin de maximiser les performances de la composition huileuse. L'huile contient également, de préférence, des composés insaponifiables dont la vitamine E et/ou le squalène. Ainsi, l'extrait huileux comprend avantageusement du squalène, des acides gras polyinsaturés en oméga-3 et/ou -6. L'extrait huileux comprend avantageusement au moins 5% en poids d'acides gras polyinsaturés en oméga-3, par rapport au poids totaux des acides gras, plus avantageusement au moins 8% en poids, encore plus avantageusement de 10% à 30% d'acides gras polyinsaturés en oméga-3, par rapport au poids totaux des acides gras. L'extrait huileux comprend avantageusement au moins 5% en poids d'acides gras polyinsaturés en oméga-6, par rapport au poids totaux des acides gras, plus avantageusement au moins 8% en poids, encore plus avantageusement de 10% à 30% d'acides gras polyinsaturés en oméga-6, par rapport au poids totaux des acides gras. L'extrait huileux comprend avantageusement de 0,1% à 2% en poids de squalène, par rapport au poids total de l'extrait huileux.

L'huile utilisée est avantageusement un mélange d'huiles végétales sources d'acides gras essentiels oméga 6 ou sources d'acides gras essentiels oméga 3 ou sources de composés insaponifiables choisies parmi l'huile d'avocat, l'huile d'amande douce, l'huile de tournesol oléique, l'huile de macadamia, l'huile de prune, l'huile de noisette, l'huile d'argan, l'huile de noyaux d'abricot, l'huile de raisin, l'huile de germe de blé, l'huile de colza, l'huile de rosier muscat, l'huile de pépins de cassis, l'huile de jojoba, l'huile de ricin, le beurre de cacao, l'huile d'échium, l'huile d'amaranthe, l'huile d'olive, l'huile de palme rouge, la cire d'olive, l'huile de bourrache, . L'huile est plus avantageusement choisie dans le groupe constitué de l'huile d'avocat, l'huile d'olive, l'huile de pépins de cassis, l'huile de rosier muscat, l'huile d'échium, la cire d'olive, l'huile d'amaranthe, l'huile de grenade, l'huile de plukenetia, l'huile de callophyle et leurs mélanges. De préférence, on utilise un mélange d'huile d'échium et d'huile d'amaranthe ou un mélange d'huile échium, d'huile d'amaranthe et d'huile d'olive.

L'huile raffinée d'échium est hautement concentrée en oméga 3 et 6, elle est hydratante, réparatrice, anti-inflammatoire et lutte contre le déficit en delta 6 désaturase des peaux matures.

L'huile d'amaranthe est riche en acide linoléique oméga 6, en vitamine E et en squalène. Elle a des propriétés émollientes, hydratantes, protectrices. Elle contribue à l'élasticité de la peau.

L'huile d'olive est riche en acide oléique, en phénols anti-oxydants et elle est source de squalène et de co-enzyme Q10. Elle contribue à nourrir et protéger la peau.

Selon une variante préférée de l'invention, l'extrait huileux de raisin Tannat et/ou Cannonau comprend une huile choisie dans le groupe comprenant l'huile raffinée d'échium, l'huile d'amaranthe et leurs mélanges, et des actifs des raisins, les raisins étant de la variété Tannat et/ou Cannoneau.

Avantageusement, le ratio massique entre les composés issus des grains de raisin Tannat et/ou Cannonau et l'huile dans l'extrait huileux est compris entre 1:0,5 et 1:10, de préférence entre 1:1 et 1:5.

L'extrait huileux est avantageusement obtenu par des procédés sans solvant (à l'exception de l'huile composant de la composition) et par un procédé impliquant uniquement des traitements thermiques et mécaniques ce qui permet de garder la répartition en acides gras et éventuellement insaponifiables originale de l'huile, utilisée en tant que vecteur, et d'assurer que les actifs du raisin, mais également les acides gras et éventuellement insaponifiables de l'huile, ne soient pas dégradés.

L'absence de solvant organique permet d'obtenir des extraits huileux exempts de traces de solvant organique. L'extrait huileux contient donc majoritairement, avantageusement de 80% à 100% en poids, par rapport au poids total de l'extrait huileux, plus avantageusement de 90% à 100% en poids, des composés issus de plantes : raisin Tannat et/ou Cannonau et huile(s) végétale(s) utilisée(s) comme solvant d'extraction.

Plusieurs procédés d'extraction existent et peuvent être utilisés. On souhaite privilégier des procédés d'extraction sans l'utilisation de solvant organique et sans transformation chimique.

Sans vouloir se limiter à ce procédé, on utilise avantageusement un procédé comprenant une étape de chauffage à haute température, avantageusement comprise entre 80°C et 200°C, et une étape de microdispersion. L'étape de chauffage est avantageusement réalisée sous micro-ondes. L'étape de microdispersion est avantageusement réalisée sous micro-ondes et/ou ultra-son. Un tel procédé a été décrit dans le brevet FR2943684 : le procédé d'extraction de composés naturels non volatils à l'aide d'un corps gras. En effet, ce procédé est adapté à l'extraction de composés naturels non volatils contenus dans une matière première solide d'origine naturelle à l'aide d'un corps gras naturel, notamment une huile ou un mélange d'huiles végétales, sans l'utilisation de solvant organique et sans transformation chimique. De plus ce procédé est conduit sous atmosphère dépourvue ou essentiellement dépourvue d'oxygène ce qui permet de limiter la dégradation oxydative de l'huile d'extraction et des bio-composés extraits des raisins Tannat et/ou Cannonau.

Ainsi, avantageusement l'extrait huileux est obtenu par un procédé comprenant les étapes successives suivantes :
a) Préparation des grains de raisins de Tannat et/ou Cannonau par déshydratation et broyage pour obtenir une forme qui soit dispersible dans une huile à une température supérieure au point de fusion de l'huile ;
b) Mélange de la poudre de raisin obtenue suite à l'étape a) avec l'huile, ou un mélange d'huiles, et imprégnation sous atmosphère dépourvue ou essentiellement dépourvue d'oxygène
c) Traitement thermique et physique du mélange obtenu suite à l'étape b) par mise en oeuvre
   - D'au moins une étape c1) de chauffage à haute température, avantageusement entre 100°C et 200°C, du mélange sur une courte durée, avantageusement inférieure ou égale à 30 minutes, et sous atmosphère dépourvue ou essentiellement dépourvue d'oxygène, puis
   - D'au moins une étape c2) de microdispersion de la matière solide dans l'huile à une température supérieure au point de fusion de l'huile et sous atmosphère dépourvue ou essentiellement dépourvue d'oxygène,
d) Récupération et filtration de l'extrait huileux de raisin Tannat et/ou Cannonau suite à l'étape c2)

Selon une caractéristique essentielle du procédé, décrit dans la demande FR 2 943 684, les étapes unitaires b) c1) et c2) sont conduites en atmosphère dépourvue ou essentiellement dépourvue d'oxygène. Ceci signifie que l'on travaille sous gaz ou atmosphère inerte ou sous vide ou vide partiel. La teneur résiduelle en oxygène doit être suffisamment basse pour ne pas provoquer de réactions d'oxydation ou de thermo-oxydation. On peut donc conduire ces étapes sous atmosphère inerte, par exemple sous azote et de préférence sous balayage continu d'azote, permettant l'extraction de l'oxygène présent ou susceptible de se former. On peut employer un réacteur clos avec une extraction continue de l'oxygène par flux d'azote. On peut aussi faire un barbotage d'azote, associé au flux d'azote, au moins au début du traitement thermique.

Les grains de raisins Tannat et/ou Cannonau sont tout d'abord deshydratés. Une préparation préalable des raisins, avantageusement des raisons verts, est avantageusement réalisée avant de débuter la déshydratation. Les grains, qui sont composés de la pellicule, des pépins et de la pulpe sont séparés de la rafle. Ils subissent ensuite un broyage mécanique qui permet de séparer la matière solide de son jus. La matière solide est ensuite placée par couches de quelques centimètres sur les grilles d'un séchoir ou d'un déshydrateur, avantageusement préréglé à une température allant de 30°C à 50°C, plus avantageusement de 40°C à 45°C. Après une durée suffisante, allant avantageusement de 24 heures à 72 heures, plus avantageusement environ 48 heures placée au séchoir ou déshydrateur, la matière solide passe d'environ 80 % d'eau à une teneur en eau idéale pour le procédé d'extraction, avantageusement de 5% à 30% en poids d'eau, plus avantageusement de 8% à 20% en poids d'eau, encore plus avantageusement de 10% à 18% en poids d'eau, par rapport au poids total.

A l'étape a), les grains de raisins Tannat et/ou Cannonau déshydratés se présentent sous une forme dissociée apte à être dispersée finement, dans l'huile ou le mélange d'huiles, et par exemple, la matière première est sous forme particulaire et de préférence pulvérulente.

Avantageusement, les grains de raisins Tannat et/ou Cannonau déshydratés sont broyés à basse température, de préférence entre -20°C et - 80°C. Par exemple cette étape peut être réalisée par congélation entre -20°C et -30°C puis broyage, par exemple à l'aide d'un broyeur à couteaux. Cette étape permet d'augmenter le rendement d'extraction par éclatement des cellules des grains des raisins Tannat ou Cannonau déshydratés, facilitant ainsi l'extraction dans l'huile de leurs actifs dont les polyphénols.

A la fin de l'étape a), la matière végétale prend avantageusement la forme d'une poudre fine de couleur marron plus ou moins foncée, elle est prête à être utilisée pour le procédé d'extraction.

Avantageusement, l'étape b) est conduite avec un ratio massique entre d'une part la poudre de raisin Tannat ou Cannonau déshydratée et broyée et d'autre part l'huile qui sert pour l'extraction de la dite poudre, compris entre 1:0,5 et 1:10, de préférence entre 1:1 et 1:5, et plus préférentiellement entre 1:1 et 1:3.

On peut avantageusement ajouter dans l'huile d'extraction un ingrédient naturel ou d'origine végétale ayant des propriétés émulsifiantes et favorisant l'extraction des composés des raisins Tannat ou Cannonau dans l'huile. Cet ingrédient peut être par exemple l'oléate de glycérol, le stéarate de glycérol, la cire de riz ou la cire de carnauba. La teneur de cet ingrédient peut être avantageusement comprise entre 1 % et 10 % et plus avantageusement entre 3% et 8 % en poids dans l'extrait huileux de raisin Tannat ou Cannonau.

L'étape b) est avantageusement conduite à une température supérieure au point de fusion de l'huile ou du mélange d'huiles utilisé, de préférence la température est comprise entre la température de fusion et la température de fusion + 20°C, et de préférence + 10°C.

La durée de l'étape b) peut être comprise entre 1 heure et 48 heures, de préférence entre 5 heures et 30 heures, mieux entre 15 heures et 28 heures, par exemple de l'ordre de 24 heures environ.

La température de l'étape c1) est avantageusement comprise entre 100°C et 200°C, de préférence entre 120°C et 190°C et plus préférentiellement entre 130°C et 170°C.

On entend par chauffage de courte durée, un traitement pour lequel la durée de la montée en température et du palier de maintien à cette température est de préférence inférieure ou égale à 30 minutes, de préférence inférieure à 20 minutes et plus préférentiellement inférieure à 15 minutes.

Tout système de chauffage thermique rapide peut être utilisé. Dans un mode de réalisation préféré, le traitement thermique est assuré par des micro-ondes. Le traitement sous micro-ondes permet de rompre les membranes cellulaires permettant une meilleure diffusion des composés des raisins Tannat ou Cannonau dans l'huile. L'étape c1) est ainsi avantageusement effectuée par un traitement micro-onde, avantageusement de forte puissance utile comprise entre 1 kWatt et 5 kWatts par kg de mélange à traiter. L'utilisation d'une source micro ondes dans un réacteur fermé permet d'atteindre en un temps court les températures souhaitées et ainsi limiter des phénomènes d'oxydation secondaire, d'autant plus que le procédé est mené en atmosphère dépourvue ou essentiellement dépourvue d'oxygène. Le chauffage à haute température permet d'accroître le pouvoir de solubilisation de l'huile et favorise le contact entre l'huile (qui joue le rôle de solvant) et les actifs, avantageusement les polyphénols, à extraire.

L'étape c2) est une étape de dispersion réalisée avantageusement sous cavitation ultrasonore. Les ultrasons permettent de rompre efficacement les membranes cellulaires des raisins, de disperser de façon homogène les composés du raisin dans l'huile. La cavitation et la dispersion sous ondes ultrasonores sont réalisées dans un réacteur fermé équipé d'un générateur d'ultrasons à basse fréquence de cavitation, notamment inférieure à 50 kHz et de préférence comprise entre 20 kHz et 30 kHz. La durée de l'étape c2) est de préférence inférieure ou égale à 30 minutes, de préférence inférieure à 20 minutes et plus préférentiellement inférieure à 15 minutes.

L'étape c2) peut être conduite à température ambiante ou à une température supérieure au point de fusion de l'huile ou du mélange d'huile utilisés. La température est avantageusement comprise entre cette température de fusion et la température de fusion +20°C, de préférence +10° C. La température ambiante (20-25°C) est parfaitement adaptée pour les huiles liquides à cette température, lorsque les étapes c1) et c2) ne sont pas conduites en même temps.

Les étapes b) c1) c2) sont avantageusement conduites en l'absence de lumière ou de tout rayonnement oxydant tels que les UV pour limiter les risques de photo-oxydation et de dégradation des molécules photosensibles contenues dans les raisins Tannat ou Cannonau.

Les étapes b) c1) c2) sont avantageusement réalisées avec agitation du mélange.

Il peut être réalisé entre chaque étape b) c1) c2) ou après la dernière étape c2), une période de maturation et de refroidissement en système fermé sous atmosphère dépourvue ou essentiellement dépourvue d'oxygène, éventuellement sous agitation douce. La durée de cette étape doit être suffisante pour une bonne diffusion des composés des raisins Tannat et/ou Cannonau dans l'huile. En particulier la durée de cette étape est comprise entre 12 heures et 48 heures, de préférence entre 18 heures et 24 heures.

L'étape d) est une étape de clarification de l'extrait huileux de raisin Tannat ou Cannonau qui est réalisée avantageusement par décantation puis par microfiltration sur une toile de porosité inférieure à 50 microns et plus avantageusement inférieure à 10 microns. Le procédé permet ainsi l'obtention d'un extrait huileux de raisin Tannat ou Cannonau se présentant sous forme de solution huileuse homogène et limpide qui soit stable dans le temps.

Suivant une caractéristique avantageuse, on ajoute après l'étape d), un composé piégeur ou réducteur d'oxygène, ou un composé permettant de régénérer sous forme réduite les tocophérols de l'huile ainsi que les composés phénoliques extraits des raisins Tannat ou Cannonau dans l'huile par le procédé ou un chélateur de métaux pro-oxydants, ces composés contribuant à améliorer la stabilité oxydative de l'extrait huileux final. Il est possible ainsi d'ajouter de la vitamine C, sous forme de palmitate d'ascorbyle, de l'acide citrique ou de l'acide lactique sous forme libre ou ester ou encore une combinaison de ces composés. Il sera ajouté une quantité individuelle de 0,01% à 1% en poids dans le mélange, préférentiellement de 0,1% à 0,5 % en poids dans le mélange. Le procédé permet d'obtenir un extrait huileux de raisin Tannat ou Cannonau non oxydé et concentré en biomolécules des raisins Tannat ou Cannonau dont les polyphénols ce qui conduit à une capacité antioxydante élevée.

Le faible niveau d'oxydation de l'huile peut s'évaluer à partir de la mesure de l'indice de peroxyde qui évalue la concentration en hydroperoxydes d'acides gras, dits composés primaires d'oxydation, pouvant se former dans l'huile en présence d'oxygène. Cette mesure est réalisée selon une méthode analytique reconnue et normalisée (NF ISO 3960). La teneur en composés phénoliques totaux peut se doser selon la méthode colorimétrique de Folin Ciocalteau.

Il est possible d'évaluer la capacité anti-oxydante totale de l'extrait huileux de raisins Tannat et/ou Cannonau obtenu par une méthode in vitro du type test ORAC - Oxygen Radical Absorbance Capacity - qui est largement utilisé et reconnu pour évaluer la capacité d'un extrait végétal à bloquer les réactions radicalaires oxydatives.

La composition selon l'invention, comprenant l'extrait huileux de raisin Tannat et/ou Cannonau, est avantageusement une composition cosmétique, dermatologique ou nutraceutique, plus avantageusement une composition cosmétique ou dermatologique.

La composition comprend en outre un extrait aqueux de raisin Tannat ou Cannonau qui est un concentrât hydrophile non fermenté stable de raisin de cépage Tannat et/ou de cépage Cannonau.

Le concentrât hydrophile a avantageusement une teneur totale en polyphénols, exprimée en équivalent épicatéchine, d'au moins 1000 ppm, plus avantageusement d'au moins 3000 ppm, encore plus avantageusement de 6 000 ppm à 100 000 ppm.

Le concentrât hydrophile a avantageusement une teneur totale en polyphénols, exprimée en équivalent oleuropéine, d'au moins 10 000 ppm, plus avantageusement d'au moins 15 000 ppm, encore plus avantageusement de 15 000 ppm à 300 000 ppm. De plus, le concentrât permet d'apporter dans la composition une quantité significative d'OPC, oligo-proanthocyanidines.

L'invention propose donc de réaliser avantageusement deux types d'extraction des raisins Tannat ou Cannonau, l'une à l'aide d'une huile et l'autre à l'aide d'une concentration du jus de raisin. La combinaison de ces deux extraits permet d'obtenir un effet de synergie et de maximiser la restitution des bénéfices de tous les constituants du raisin (peau, chair et pépins). La combinaison d'une composition huileuse, comprenant les actifs de raisin Tannat et/ou Cannonau, et d'un concentrât hydrophile de raisin Tannat et/ou Cannonau, chacun étant spécifiquement enrichi en actifs tels que les polyphénols, permet de maximiser l'efficacité des compositions en étant plus proche de la composition d'actifs initialement présents dans le raisin. Ainsi, le concentrât permet d'apporter dans la composition une quantité significative d'OPC.

Avantageusement, le ratio massique (extrait huileux) : (concentrât hydrophile) varie de 10 :1 à 1 :10, plus avantageusement de 10 :1 à 1 : 1, encore plus avantageusement de 6 :1 à 2 :1. Ce ratio massique peut être de 4 :1

Ainsi, la composition comprend avantageusement à titre d'actif l'association de l'extrait huileux et du concentrât hydrophile. Dans une variante préférée de l'invention, chaque composant, que l'on appelle aussi extrait de raisin, est dédié à une phase d'une émulsion eau-dans huile (E/H) ou huile-dans-eau (H/E) afin de coupler les actions antioxydantes, fluidifiantes, hydratantes de chacun des extraits.

L'extrait huileux et le concentrât hydrophile sont deux extraits complémentaires et synergiques issus des grains de raisin, Tannat et/ou Cannonau, pour apporter une activité maximale au produit cosmétique, dermatologique ou nutraceutique.

On a constaté, d'une manière surprenante, que l'effet antioxydant, mesurée par le test ORAC, d'une composition comprenant l'extrait huileux et le concentrât hydrophile, est supérieur à l'effet antioxydant attendu résultant de la somme de l'effet antioxydant, mesurée par le test ORAC, pour l'extrait huileux et pour le concentrât hydrophile. Les deux extraits, utilisés ensemble dans une composition, présentent donc un effet antioxydant synergique.

La teneur en extrait huileux, dans la composition, varie avantageusement de 0,1% à 99% en poids, par rapport au poids total de la composition. Avantageusement, dans une composition cosmétique ou dermatologique, elle varie de 0,1% à 30% en poids, plus avantageusement de 0,2% à 10% en poids.

La teneur en concentrât hydrophile, dans la composition, varie avantageusement de 0,02% à 99% en poids, par rapport au poids total de la composition. Avantageusement, dans une composition cosmétique ou dermatologique, elle varie de 0,02% à 30% en poids, plus avantageusement de 0,02 à 10% en poids.

La teneur totale en actif antioxydant selon l'invention (à savoir la composition huileuse et/ou le concentrât hydrophile), dans la composition, varie avantageusement de 0,05% à 99% en poids, par rapport au poids total de la composition.

Lorsque la composition est une composition cosmétique ou dermatologique, la teneur en actif antioxydant selon l'invention, dans la composition, varie avantageusement de 0,05% à 50% en poids, plus avantageusement de 0,1% à 30% en poids, par rapport au poids total de la composition.

Lorsque la composition est une composition nutraceutique de type aliment, la teneur en actif antioxydant selon l'invention, dans la composition, varie avantageusement de 1% à 99 % en poids, par rapport au poids total de la composition.

Lorsque la composition est une composition nutraceutique de type complément alimentaire, la teneur en actif antioxydant selon l'invention, dans la composition, varie avantageusement de 5% à 99% en poids, par rapport au poids total de la composition.

Le concentrât hydrophile est un jus de raisin concentré qui peut être obtenu par tout procédé adapté.

Pour le concentrât hydrophile, on utilise avantageusement un procédé comprenant une étape de concentration conduite à haute température et sous l'action de micro-ondes. L'activation énergétique sous micro-ondes permet une stérilisation du concentrât et réduit le risque de fermentation possible. De plus, une grande partie de la phase aqueuse est éliminée par évaporation lors de cette étape sous micro-ondes, le produit ainsi obtenu est concentré en matières actives, en particulier en polyphénols. Le procédé comprend également, de manière avantageuse, suite à l'étape de concentration, une étape de filtration. Cette étape de filtration met avantageusement en oeuvre un filtre ayant une porosité variant de 0,1 mm à 3 mm, plus avantageusement de 0,2 à 2 mm.

En particulier, le concentrât hydrophile de raisin est obtenu par un procédé comprenant les étapes successives suivantes :
i. Préparation d'un jus de grains de raisins ;
ii. Chauffage à une température comprise entre 50°C et 200°C du jus obtenu suite à l'étape i. ;
iii. Récupération et filtration du concentrât hydrophile.

A l'étape i), le jus de raisin peut être obtenu par tout procédé connu de l'homme du métier. Le plus souvent, le jus est récupéré après pressage des grains de raisin Tannat et/ou Cannonau. Il est conservé, avant utilisation, à basse température, avantageusement d'environ -18°C ± 2°C, afin de préserver tous ces composants et d'éviter toute fermentation.

Le jus est avantageusement mélangé sous agitation afin d'obtenir un mélange homogène. On préfère, tel que cela a été décrit précédemment, utiliser les raisins récoltés avant véraison.

La température de l'étape ii) est avantageusement comprise entre 50°C et 150°C et plus préférentiellement entre 80°C et 130°C.

Le chauffage est réalisé le temps nécessaire pour obtenir une composition homogène, d'aspect liquide à pâteux, contenant moins de 80% en poids d'eau, par rapport au poids total de la composition, avantageusement moins de 30% en poids d'eau, plus avantageusement de 1% à 30% en poids d'eau.

En fonction de la quantité de jus à traiter et de la puissance de chauffe, cette étape peut durer de 5 heures à 15 heures, plus avantageusement de 7 heures à 12 heures. A titre d'exemple à une puissance de 4000 watts sur une masse de 1 kg de jus de raisin Tannat ou Cannonau, il faut environ 10 heures et sur une masse de 10 kg environ 20 heures.

Tout système de chauffage thermique rapide peut être utilisé. Dans un mode de réalisation préféré, le traitement thermique est assuré par des micro-ondes. L'étape ii) est ainsi avantageusement effectuée par un traitement micro-onde, avantageusement on emploie des micro-ondes de puissance utile comprise entre 300 Watts et 6 000 Watts, plus avantageusement de 500 Watts à 4 000 Watts, par kg de mélange à traiter. L'utilisation d'une source microondes dans un réacteur fermé permet d'atteindre en un temps court les températures souhaitées.

Cette étape ii) peut être répétée plusieurs fois. A la fin d'une étape ii), avant nouvelle itération, on peut observer une période de repos du jus en cours de traitement à température ambiante pendant avantageusement 12 heures à 24 heures. Avantageusement, cette étape ii) est réalisée une seule fois, sans réitération, et avec une agitation régulière du jus.

L'étape ii) peut être réalisée avec ou sans agitation du mélange et préférentiellement avec agitation.

Lors de l'étape iii), le concentrât hydrophile peut être récupéré par filtration. Cette étape de filtration met avantageusement en oeuvre un filtre, tel qu'un filtre en polypropylène non tissé, ayant une porosité variant de 0,1 mm à 3 mm, plus avantageusement de 0,2 à 2 mm. Après filtration, le concentrât est homogène, semi-fluide et est riche en polyphénols natifs de raisin Tannat ou Cannonau.

Le procédé comprend, suite à l'étape iii), une étape supplémentaire iv) consistant en l'ajout d'un conservateur microbien, antifongique ou antibactérien, tel que la chlorphénésine ou tout autre conservateur microbien compatible avec l'application visée. Sa présence empêche tout développement microbien dans le concentrât. La teneur en conservateur varie avantageusement de 0,10 à 0,30 % en poids, par rapport au poids total du concentrât, plus avantageusement entre 0,18 et 0,28 % en poids.

Le procédé permet d'obtenir un concentrât stabilisé de raisin Tannat ou Cannonau enrichi en polyphénols, ceci grâce à la mise en oeuvre d'un procédé qui utilise une concentration à haute température, avantageusement sous micro-ondes, une étape de filtration avantageusement à 0,1 mm - 3 mm, et, dans une variante, par l'ajout d'un conservateur.

Le concentrât hydrophile de jus de raisin Tannat ou Cannonau est stable physiquement et microbiologiquement. Ainsi, le concentrât reste homogène, il ne fermente pas et on n'observe pas de développement bactérien.

La composition comprend avantageusement en outre un ou plusieurs excipients adaptés à une utilisation cosmétique, dermatologique ou nutraceutique. A titre d'exemple, on peut citer :
- les stabilisants, de préférence les stabilisants naturels, en particulier choisis dans le groupe constitué de vitamine C liposoluble, d'acide carnosique, et de tocophérol(s) ;
- l'acide ascorbique libre, sel ou ester, l'acide citrique ou lactique libre ou ester, ou des lécithines ;
- les conservateurs, tels que la chlorphénésine.

On peut également trouver dans la composition des molécules utilisées dans les procédés d'extraction, en particulier des molécules amphiphiles, telles que le stéarate de glycérol, qui permettent de stabiliser les colloïdes formés lors de la lipophilisation des polyphénols au sein de la composition huileuse.

La composition peut également comprendre tout autre actif, en particulier cosmétique, dermatologique ou nutraceutique, approprié. Avantageusement, la composition comprend à titre d'actif anti-oxydant l'association de l'extrait huileux et/ou le concentrât hydrophile.

Avantageusement la composition est appliquée par voie topique ou orale, plus avantageusement par voie topique.

La composition peut également être destinée à être utilisée sur tissus et fibres imprégnés.

L'invention a aussi pour objet une composition selon l'invention, comprenant l'extrait huileux et le concentrât hydrophile, pour son utilisation en tant qu'agent antioxydant.

Les extraits de raisin Tannat ou Cannonau selon l'invention, l'extrait huileux et le concentrât hydrophile, présentent un pouvoir antioxydant qui permet leur utilisation pour lutter contre le stress oxydatif, en particulier pour piéger les radicaux libres. L'invention a aussi pour objet une composition selon l'invention pour son utilisation pour lutter contre le stress oxydatif, en particulier pour piéger les radicaux libres.

Plus particulièrement, l'extrait huileux est riche en flavonoïdes et en acides phénoliques. Cet extrait permet tout particulièrement de conférer une action hydratante, de restructuration de la peau et des phanères, fluidifiante, antiride, antioxydante.

Le concentrât hydrophile est riche en OPC et tannin. Ce concentrât permet tout particulièrement de conférer une action antioxydante.

Avantageusement, la composition est destinée à retarder le vieillissement cutané. Cela peut se traduire par un retard de l'apparition des rides, des ridules, un raffermissement du derme, une protection de l'ADN contre les dommages induits par les radicaux libres.

Avantageusement, la composition est destinée à améliorer l'aspect de la peau et/ou traiter les effets de l'exposition au soleil.

On entend désigner par le terme " traitement " ou " traiter ", et toutes ses déclinaisons, le traitement curatif ou préventif. La composition selon l'invention peut ainsi être utilisé chez un sujet désireux de se protéger du stress oxydatif et/ou voulant diminuer les effets néfastes sur sa peau ou les phanères du stress oxydatif. La composition selon l'invention peut aussi être utilisée pour retarder ou ralentir la progression ou prévenir une progression plus en avant des effets du stress oxydatif.

Cette utilisation peut également être pour la préparation d'une composition pharmaceutique destinée à traiter le stress oxydant et/ou traiter les effets de l'exposition aux rayonnements ionisants ou solaires, et/ou prévenir le vieillissement notamment de la peau, des cheveux, des cils, des sourcils et/ou des ongles, et/ou traiter les effets de l'utilisation de certains médicaments générateurs de radicaux libres.

Selon un autre aspect, l'invention concerne la composition pour son utilisation en tant qu'agent antioxydant, avantageusement dans une méthode de traitement dermatologique du stress oxydatif sur la peau, comprenant l'administration topique ou orale de la composition selon l'invention telle que décrite ci-dessus à une personne en ayant besoin.

La présente invention a pour objet l'utilisation de la composition pour la préparation de préparations alimentaires, d'alicaments, ou de compositions pharmaceutiques destinés à la prévention ou au traitement du stress oxydant.

L'invention a aussi pour objet un procédé sans solvant de préparation d'un concentrât hydrophile de raisin Tannat ou Cannonau stabilisé physiquement et microbiologiquement comprenant les étapes successives suivantes :
i. Préparation d'un jus de grains de raisins Tannat ou Cannonau ;
ii. Chauffage micro-ondes, à une température comprise entre 80°C et 130°C, du jus obtenu suite à l'étape i. ;
iii. Récupération et filtration du concentrât hydrophile.
iv. Ajout au concentrât d'un conservateur

Ces étapes sont telles que définies précédemment.

La matière première utilisée est avantageusement le jus obtenu par pressage de grains de raisin de cépage Tannat et/ou Cannonau. On préfère, tel que cela a été décrit précédemment, utiliser les raisins récoltés avant véraison.

L'invention a également pour objet un concentrât hydrophile de grains de raisin de cépage Tannat ou Cannonau, obtenu par le procédé décrit précédemment, comprenant au moins 0,5 g/100g d'OPC exprimés en éq. Procyanidine B2. Ce concentrât est stable physiquement et microbiologiquement.

Les exemples qui suivent permettent d'illustrer l'invention. Sauf indication contraire, les pourcentages sont exprimés en poids.

### Exemple 1 : préparation d'un extrait huileux de raisin vert Tannat et d'un concentrât hydrophile de raisin vert Tannat

### Récolte du raisin :

Les études expérimentales ont été menées avec le raisin Tannat dans deux états de maturité, l'un vert et l'autre mur donc noir.

Le raisin vert de la variété Tannat a été sélectionné pour sa richesse en polyphénols et en procyanidines.

### Préparation des matières d'oeuvre:

Une préparation préalable des raisins verts est réalisée avant de débuter la déshydratation. Les grains, qui sont composés de la pellicule, des pépins et de la pulpe sont séparés de la rafle. Ils subissent ensuite un pressage mécanique qui permet de séparer la matière solide de son jus.

La matière solide est placée par couches de quelques centimètres sur les grilles du déshydrateur préréglé à 40°C. Après environ 48 h, la matière solide passe d'environ 80 % d'eau à 12 % d'eau, teneur en eau idéale pour le procédé d'extraction. Les raisins déshydratés sont ensuite stockés à -80°C puis broyés dans un broyeur à couteaux. On obtient une poudre fine de couleur marron-jaune.

Le jus obtenu au cours du pressage des grains est un mélange inhomogène d'eau et de particules de raisin, de couleur verte. Ce jus est conservé à basse température afin de préserver tous ces composants et d'éviter toute fermentation.

### Procédé d'extraction

### L'extrait huileux

L'extrait huileux de raisin vert Tannat est obtenu avec un mélange de 72 % d'huile raffinée d'échium contenant au moins 24 % d'acide alpha-linolénique oméga 3, 23 % d'huile vierge d'amaranthe contenant au moins 45 % d'acide linoléique oméga 6 et 4 % de squalène et 5 % de stéarate de glycérol.

L'extraction est réalisée sur 470 g de poudre de raisin vert tannat et 1410 g du mélange huile d'échium + huile d'amaranthe + stéarate de glycérol, soit un ratio 1 :3 (m/m) poudre de raisin vert Tannat / vecteur huileux.

L'étape b) est réalisée à température ambiante pendant 24 h sous azote. L'étape c1) sous microondes est réalisée pendant 8 min à 145°C sous azote. L'étape c2) sous ultrasons est réalisée pendant 15 min à température ambiante sous azote. L'étape d) est réalisée par microfiltration centrifuge sur toile filtrante de porosité 5 microns. Il est ajouté dans l'extrait huileux de raisin vert Tannat, 0,4 % en poids d'extrait de romarin et 0,1 % en poids de mélange de tocophérols.

L'extrait huileux de raisin vert Tannat est homogène, vert clair, avec une note olfactive de verdure, se rapprochant du raisin, peu intense. L'extrait huileux de raisin vert Tannat contient moins de 0,4 % d'eau et présente un indice de peroxyde inférieur à 15 méqO2/kg.

### Le concentrât hydrophile

A partir de 33 kg de raisins verts Tannat, on obtient 10 kg de jus de raisin vert. Le jus de raisin vert Tannat est concentré dans un réacteur sous microondes à 115 °C pour obtenir 2 kg de concentrat hydrophile de raisin vert Tannat. Il est ajouté dans le concentrat hydrophile de raisin vert Tannat 0,25 % de chlorphénésine.

Le concentrat hydrophile obtenu à partir du jus de raisin vert Tannat est stable, d'odeur fruitée et de couleur vert sombre.

Des contrôles microbiologiques ont été effectués pour vérifier la stabilité microbiologique du concentrat hydrophile de raisin vert Tannat en mesurant les moisissures et bactéries sur bi-géloses après 2 jours à 37°C et 2 jours à température ambiante. Aucun développement microbien n'a été détecté.

### Exemple 2 : Teneur en polyphénols d'un extrait huileux de raisin vert Tannat et d'un concentrât hydrophile de raisin vert ou noir Tannat

Les extraits de raisin vert Tannat ont été préparés dans les conditions de l'exemple 1.

A titre comparatif, un concentrat hydrophile de raisin noir (mûr) Tannat, un extrait huileux de raisins verts de table, un concentrat hydrophile de raisins verts de table ont été réalisés dans les conditions de l'exemple 1. Les extraits ainsi obtenus ont été caractérisés notamment la teneur en polyphénols totaux a été mesurée par spectrophotométrie selon la méthode de Folin.

**Tableau 1**

| | Indice de Peroxyde | Teneur en phénols totaux en épicatéchine équivalent |
|---|---|---|
| Extrait huileux de raisin vert Tannat | 11,1 méqO2/kg | 875 mg / kg |
| Concentrat hydrophile de raisin vert Tannat | / | 62 110 mg / kg |
| Concentrat hydrophile de raisin noir Tannat | / | 6 500 mg / kg |

Les résultats des teneurs en phénols des extraits confirment l'intérêt de produire un concentrat hydrophile à partir de raisins verts de la variété Tannat.

### Exemple 3 : Evaluation de l'activité antioxydante d'un extrait huileux de raisin vert Tannat et d'un concentrât hydrophile de raisin vert ou noir Tannat

Les extraits de raisin vert ou noir Tannat ont été préparés dans les conditions de l'exemple 1.

Les extraits obtenus ont été évalués seuls et en mélange pour leurs propriétés antioxydantes en utilisant le test in vitro ORAC (Oxygen Radical Absorbance Capacity). Ce test permet d'évaluer la capacité à piéger les radicaux libres, signe d'une activité anti-âge. Ce test permet d'évaluer la capacité d'un extrait végétal à bloquer les réactions radicalaires oxydatives. Il mesure les dommages causés par des radicaux libres sur un marqueur fluorescent, ce qui se traduit par une baisse de l'intensité de la fluorescence mesurée en spectrophotométrie à fluorescence. La présence d'antioxydants dans un produit à tester empêche les radicaux libres d'agir sur le composé fluorescent. La capacité anti-oxydante de l'actif est comparée à celle d'un équivalent de la vitamine E, le Trolox ^{®}. Les résultats sont exprimés en Trolox^{®} équivalent (TE) par unité de poids d'échantillon.

**Tableau 2**

| | Teneur en phénols totaux en épicatéchine équivalent | Valeur ORAC |
|---|---|---|
| Extrait huileux de raisin vert Tannat | 875 mg / kg | 18 386 µmole TE /kg |
| Concentrat hydrophile de raisin vert Tannat | 62 110 mg / kg | 515 868 µmole TE /kg |
| Concentrat hydrophile de raisin noir Tannat | 6500 mg /kg | 47 650 µmole TE /kg |

Les résultats du test ORAC confirment l'intérêt de produire un concentrat hydrophile à partir de raisins verts de la variété Tannat.

### Exemple 4 : Evaluation de l'activité antioxydante d'un extrait huileux de raisin vert Tannat et d'un concentrât hydrophile de raisin vert ou noir Tannat et mise en évidence de l'effet antioxydant synergique

On prépare une crème cosmétique A contenant 10% d'extrait huileux de raisin vert Tannat et 2,5 % de concentrat hydrophile de raisin vert Tannat préparés selon l'exemple 1.

On prépare une deuxième crème cosmétique B contenant 10% d'extrait huileux de raisin vert Tannat et 2,5 % de concentrat hydrophile de raisin noir Tannat préparés selon l'exemple 1.

Les valeurs ORAC des deux crèmes A et B sont d'une part calculées à partir des valeurs ORAC mesurées pour les extraits seuls et d'autre part mesurées expérimentalement.

**Tableau 3 : valeurs ORAC**

| | Valeur ORAC Théorique | Valeur ORAC mesurée |
|---|---|---|
| CREME A | 14 735 µmole TE /kg | 19 895 µmole TE /kg |
| CREME B | 3 030 µmole TE /kg | 5 180 µmole TE /kg |

Ces résultats montrent très clairement que :
- Tous les extraits de raisin Tannat obtenus par l'invention ont un pouvoir antiradicalaire ,
- Le concentrat hydrophile de raisin vert Tannat est plus efficace que celui de raisin noir Tannat pour protéger du stress oxydant,
- Une synergie existe entre l'extrait huileux de raisin vert Tannat et le concentrat hydrophile de raisin Tannat avec une amélioration de +30 % du pouvoir antioxydant lorsqu'on combine le concentrât hydroactif de raisin vert à l'extrait huileux de raisin vert et de +70% lorsqu'on combine le concentrat hydroactif de raisin noir à l'extrait huileux de raisin vert.

### Exemple 5 : Evaluation de l'action sur le collagène et sur les sirtuines (Sirt-1) d'un extrait huileux de raisin vert Tannat et d'un concentrât hydrophile de raisin vert Tannat

L'étude a pour objet l'évaluation d'une composition comprenant l'extrait huileux et le concentrât selon l'invention :
- sur la modulation de l'activité de la sirtuine 1 (Sirt-1) dans des cellules cultivées (cellules de fibroblaste humaines) après exposition à un stress (H₂O₂). Les cellules sont soumises à un stress (H₂O₂) puis traitées avec les compositions C ou D pendant 48 heures ou 72 heures. A la fin de cette période, on dose (kit ELISA) dans les cellules l'activité des sirtuines (Sirt-1). Les résultats sont comparés au dosage des sirtuines (Sirt-1) dans les cellules non traitées.
- Sur la capacité des compositions à stimuler la synthèse *ex novo* du collagène. On travaille sur des cellules de fibroblaste humaines. Les cellules sont mises en contact avec les compositions et on mesure par colorimétrie la synthèse ex novo du collagène. Le réactif colorant est un colorant anionique porteur de chaînes latérales acide sulfonique qui réagit avec les chaînes latérales des acides aminés basiques présents dans le collagène et se lie spécifiquement à la structure hélicoïdale [Gly-X-Y]ₙ présente dans tous les collagènes.

On prépare une crème cosmétique C contenant 0,5% d'extrait huileux de raisin vert Tannat et 0,13 % de concentrat hydrophile de raisin vert Tannat préparés selon l'exemple 1.

On prépare une deuxième crème cosmétique D contenant 0,25% d'extrait huileux de raisin vert Tannat et 0,06 % de concentrat hydrophile de raisin vert Tannat préparés selon l'exemple 1.

Les résultats sont reportés dans les tableaux suivants:

**Tableau 4 : effet sur le collagène**

| Traitement | Temps | % variation vs Contrôle-(contrôle négatif) |
|---|---|---|
| **Composition C** | 48 h | +3,7% |
| **Composition C** | 72 h | +20.7% |
| **Composition D** | 48 h | +8.3% |
| **Composition D** | 72 h | +16.9% |

### SIRTUIN TEST:

**Tableau 5: effet sur les sirtuines 1 (Sirt-1)**

| Traitement | Temps | % variation vs Contôle (contrôle positif : H₂O₂) |
|---|---|---|
| **Composition C** | 72 h | +18,4% |
| **Composition D** | 72 h | +21.7% |

On constate que les extraits selon l'invention permettent de stimuler la synthèse *ex novo* du collagène et d'augmenter les teneurs en sirtuine 1 (Sirt-1).

## Revendications

1. Composition, avantageusement cosmétique, dermatologique ou nutraceutique, comprenant (a) un extrait huileux de raisin de cépage Tannat et/ou de cépage Cannonau et (b) un concentrat hydrophile de raisin de cépage Tannat et/ou de cépage Cannonau et (c) éventuellement un excipient dermatologiquement ou nutraceutiquement acceptable, ledit extrait huileux ayant un indice de péroxyde inférieur à 20 méqO₂ par kg d'extrait huileux, et ledit extrait huileux étant obtenu par mélange des grains de raisins de cépage Tannat et/ou de cépage Cannonau et d'au moins une huile puis extraction physique des actifs non volatils lipophiles des grains de raisin vers ladite huile par un procédé impliquant uniquement des traitements thermiques et mécaniques, sans impliquer d'autre solvant.

2. Composition selon la revendication 1, **caractérisée en ce que** l'huile utilisée pour la préparation de la composition huileuse est un mélange d'huile d'échium et d'huile d'amaranthe ou d'huile d'échium, d'huile d'amaranthe et d'huile d'olive.

3. Composition selon l'une quelconque des revendications précédentes, **caractérisée en ce que** l'extrait huileux comprend du squalène, des acides gras polyinsaturés en oméga-3 et/ou -6.

4. Composition selon l'une quelconque des revendications précédentes, **caractérisée en ce que** l'extrait huileux est obtenu par un procédé comprenant les étapes successives suivantes :
a) Préparation des grains de raisins de Tannat et/ou Cannonau par déshydratation et broyage pour obtenir une forme qui soit dispersible dans une huile à une température supérieure au point de fusion de l'huile ;
b) Mélange de la poudre de raisin obtenue suite à l'étape a) avec l'huile, ou un mélange d'huiles, et imprégnation sous atmosphère dépourvue ou essentiellement dépourvue d'oxygène
c) Traitement thermique et physique du mélange obtenu suite à l'étape b) par mise en oeuvre
• D'au moins une étape c1) de chauffage à haute température, avantageusement entre 100°C et 200°C, du mélange sur une courte durée, avantageusement inférieure ou égale à 30 minutes, et sous atmosphère dépourvue ou essentiellement dépourvue d'oxygène, puis
• D'au moins une étape c2) de microdispersion de la matière solide et éventuellement de rupture des cellules de la matière première, dans l'huile à une température supérieure au point de fusion de l'huile et sous atmosphère dépourvue ou essentiellement dépourvue d'oxygène,
d) Récupération et filtration de l'extrait huileux de raisin Tannat et/ou Cannonau suite à l'étape c2)

5. Composition selon la revendication 4 **caractérisée en ce que** l'étape c1) est effectuée par un traitement micro-onde, avantageusement on emploie des micro-ondes de forte puissance utile comprise entre 1 kWatt et 5 kWatts par kg de mélange à traiter.

6. Composition selon l'une quelconque des revendications 4 à 5, **caractérisée en ce que** l'étape c2) comprend un traitement par cavitation ultrasonore ; en particulier la fréquence de cavitation est inférieure à 50 kHz, de préférence comprise entre 20 kHz et 30 kHz.

7. Composition selon l'une quelconque des revendications précédentes , **caractérisée en ce que** le concentrât hydrophile de raisin est obtenu par un procédé comprenant les étapes successives suivantes :
i. Préparation d'un jus de grains de raisins Tannat et/ou Cannonau ;
ii. Chauffage à une température comprise entre 50°C et 200°C du jus obtenu suite à l'étape i. ;
iii. Récupération du concentrât hydrophile
iv. Avantageusement, ajout au concentrât d'un conservateur, tel que la chlorphénésine.

8. Composition selon la revendication 7, **caractérisée en ce que** l'étape ii) est effectuée par un traitement micro-onde, avantageusement on emploie des micro-ondes de forte puissance utile comprise entre 300 Watts et 6 000 Watts par kg de mélange à traiter.

9. Composition selon l'une quelconque des revendications précédentes, **caractérisée en ce que** le ratio massique (extrait huileux) : (concentrât hydrophile) varie de 10 :1 à 1 :10.

10. Composition selon l'une quelconque des revendications précédentes, **caractérisée en ce qu'**elle comprend en outre un ou plusieurs excipients choisis parmi la vitamine C liposoluble, l'acide carnosique, le tocophérol, l'acide ascorbique libre, sel ou ester, l'acide citrique ou lactique libre ou ester, des conservateurs naturels tels que la chlorephénésine.

11. Composition selon l'une quelconque des revendications précédentes pour son utilisation en tant qu'agent antioxydant, avantageusement pour prévenir le stress oxydatif.

12. Procédé sans solvant de préparation d'un concentrât hydrophile de raisin stabilisé physiquement et microbiologiquement comprenant les étapes successives suivantes :
i. Préparation d'un jus de grains de raisins Tannat et/ou Cannonau ;
ii. Chauffage micro-ondes, à une température comprise entre 80°C et 130°C, du jus obtenu suite à l'étape i. ;
iii. Récupération du concentrât hydrophile.
iv. ajout au concentrât d'un conservateur, tel que la chlorphénésine.

13. Concentrât hydrophile de grains de raisin, de cépage Tannat ou Cannonau, obtenu par le procédé selon la revendication 12, comprenant au moins 0,5 g/100g d'OPC exprimés en éq. Procyanidine.

## Patentansprüche

1. Zubereitung, vorzugsweise kosmetische, dermatologische oder nutrazeutische, umfassend (a) einen Ölextrakt aus Trauben der Tannat-Rebe und/oder der Cannonau-Rebe und (b) ein hydrophiles Konzentrat aus Trauben der Tannat-Rebe und/oder der Cannonau-Rebe und (c) gegebenenfalls einen dermatologisch oder nutrazeutisch akzeptablen Hilfsstoff, wobei der Ölextrakt eine Peroxidzahl von weniger als 20 mEqO₂ hat und wobei dieser Ölextrakt durch Mischen der Kerne von Trauben der Tannat-Rebe und/oder der Cannonau-Rebe und mindestens einem Öl gewonnen wird, gefolgt von der physikalischen Extraktion der nicht flüchtigen lipophilen Wirkstoffe der Traubenkerne in das Öl durch ein Verfahren, das nur thermische und mechanische Behandlungen einsetzt, ohne ein anderes Lösungsmittel einzusetzen.

2. Zubereitung nach Anspruch 1, **dadurch gekennzeichnet, dass** das zur Herstellung der Ölzubereitung verwendete Öl ein Gemisch aus Echiumöl und Amaranthöl oder Echiumöl, Amaranthöl und Olivenöl ist.

3. Zubereitung nach einem der vorhergehenden Ansprüche, **dadurch gekennzeichnet, dass** der Ölextrakt Squalen, mehrfach ungesättigte Omega-3- und/oder Omega-6-Fettsäuren umfasst.

4. Zubereitung nach einem der vorhergehenden Ansprüche, **dadurch gekennzeichnet, dass** der Ölextrakt durch ein Verfahren gewonnen wird, das die folgenden aufeinanderfolgenden Schritte umfasst:
a) Vorbereiten der Tannat- und/oder Cannonau-Traubenkerne durch Entwässern und Zerkleinern, um eine Form zu erhalten, die in einem Öl bei einer Temperatur über dem Schmelzpunkt des Öls dispergierbar ist;
b) Mischen des aus Schritt a) gewonnenen Traubenpulvers mit dem Öl oder einem Ölgemisch und Imprägnieren unter Atmosphäre ohne oder im Wesentlichen ohne Sauerstoff;
c) thermisches und physikalisches Behandeln des aus Schritt b) gewonnenen Gemischs durch Durchführen
• von mindestens einem Heizschritt c1) bei hoher Temperatur, in vorteilhafter Weise zwischen 100 °C und 200 °C, des Gemischs während einer kurzen Dauer, in vorteilhafter Weise von weniger oder gleich 30 Minuten, und unter Atmosphäre ohne oder im Wesentlichen ohne Sauerstoff, dann
• von mindestens einem Schritt c2) der Mikrodispersion des Feststoffs und gegebenenfalls des Aufschließens der Zellen des Rohstoffs im Öl bei einer Temperatur oberhalb des Schmelzpunkts des Öls und unter Atmosphäre ohne oder im Wesentlichen ohne Sauerstoff,
d) Rückgewinnen und Filtrieren des Ölextrakts der Tannat- und/oder Cannonau-Trauben aus Schritt c2).

5. Zubereitung nach Anspruch 4, **dadurch gekennzeichnet, dass** der Schritt c1) durch eine Mikrowellenbehandlung erfolgt, wobei in vorteilhafter Weise Mikrowellen mit einer hohen Nutzleistung zwischen 1 kWatt und 5 kWatt je kg zu behandelnde Mischung verwendet werden.

6. Zubereitung nach einem der Ansprüche 4 bis 5, **dadurch gekennzeichnet, dass** Schritt c2) eine Ultraschall-Kavitationsbehandlung umfasst; wobei die Kavitationsfrequenz insbesondere unter 50 kHz, vorzugsweise zwischen 20 kHz und 30 kHz, liegt.

7. Zubereitung nach einem der vorhergehenden Ansprüche, **dadurch gekennzeichnet, dass** das hydrophile Traubenkonzentrat durch ein Verfahren gewonnen wird, das die folgenden aufeinanderfolgenden Schritte umfasst:
i. Zubereiten eines Saftes aus Tannat- und/oder Cannonau-Traubenkernen;
ii. Erhitzen des aus Schritt i. gewonnenen Saftes auf eine Temperatur zwischen 50 °C und 200 °C;
iii. Rückgewinnen des hydrophilen Konzentrats;
iv. vorzugsweise Zugabe eines Konservierungsmittels wie Chlorphenesin zum Konzentrat.

8. Zubereitung nach Anspruch 7, **dadurch gekennzeichnet, dass** der Schritt ii) durch eine Mikrowellenbehandlung durchgeführt wird, wobei in vorteilhafter Weise Mikrowellen mit einer hohen Nutzleistung zwischen 300 Watt und 6000 Watt je kg zu behandelnde Mischung verwendet werden.

9. Zubereitung nach einem der vorhergehenden Ansprüche, **dadurch gekennzeichnet, dass** das Massenverhältnis (Ölextrakt): (hydrophiles Konzentrat) von 10:1 bis 1:10 schwankt.

10. Zubereitung nach einem der vorhergehenden Ansprüche, **dadurch gekennzeichnet, dass** sie ferner einen oder mehrere Hilfsstoffe umfasst, die aus fettlöslichem Vitamin C, Karnosinsäure, Tocopherol, freier Ascorbinsäure, Salz oder Ester, freier Zitronen- oder Milchsäure oder Ester, natürlichen Konservierungsstoffen wie Chlorphenesin ausgewählt sind.

11. Zubereitung nach einem der vorhergehenden Ansprüche für ihre Verwendung als Antioxidans, in vorteilhafter Weise zur Vorbeugung von oxidativem Stress.

12. Lösemittelfreies Verfahren zur Herstellung eines physikalisch und mikrobiologisch stabilisierten hydrophilen Traubenkonzentrats, das die folgenden aufeinanderfolgenden Schritte umfasst:
i. Zubereiten eines Saftes aus Tannat- und/oder Cannonau-Traubenkernen;
ii. Erhitzen des Saftes aus Schritt i. in der Mikrowelle auf eine Temperatur zwischen 80 °C und 130 °C;
iii. Rückgewinnen des hydrophilen Konzentrats;
iv. Zugabe eines Konservierungsmittels wie Chlorphenesin zum Konzentrat.

13. Hydrophiles Konzentrat aus Traubenkernen der Tannat- oder Cannonau-Rebe, gewonnen durch das Verfahren nach Anspruch 12, umfassend mindestens 0,5 g/100 g OPC, ausgedrückt in Procyanidin-Äquivalent.

## Claims

1. A composition, advantageously cosmetic, dermatological or nutraceutical, comprising (a) an oily extract of Tannat variety grape and/or Cannonau variety grape and (b) a hydrophilic concentrate of Tannat variety grape and/or Cannonau variety grape and (c) optionally a dermatologically or nutraceutically acceptable excipient, said oily extract having a peroxide value of less than 20 meqO₂ per kg of oily extract, and said oily extract being obtained by mixing the Tannat variety and/or Cannonau variety grape berries and at least one oil and then physically extracting the lipophilic non-volatile active ingredients from the grape berries to said oil by a method involving only thermal and mechanical treatments, without involving any other solvent.

2. The composition according to claim 1, **characterised in that** the oil used for preparing the oily composition is a mixture of echium oil and amaranth oil or echium oil, amaranth oil and olive oil.

3. The composition according to any of the preceding claims, **characterised in that** the oily extract comprises squalene, omega-3 and/or -6 polyunsaturated fatty acids.

4. The composition according to any of the preceding claims, **characterised in that** the oily extract is obtained by a method comprising the following successive steps:
a) Preparing the Tannat and/or Cannonau grape berries by dehydration and crushing to obtain a form that is dispersible in an oil at a temperature above the melting point of the oil;
b) Mixing the grape powder obtained following step a) with the oil, or a mixture of oils, and impregnating in an atmosphere devoid or essentially devoid of oxygen
c) Thermally and physically treating the mixture obtained following step b) by implementing
• At least one step c1) of heating to a high temperature, advantageously between 100°C and 200°C, the mixture over a short period of time, advantageously less than or equal to 30 minutes, and in an atmosphere devoid or essentially devoid of oxygen, then
• At least one step c2) of microdispersing the solid and possibly breaking the cells of the raw material, in the oil at a temperature above the melting point of the oil and in an atmosphere devoid or essentially devoid of oxygen,
d) Recovering and filtering the oily extract of Tannat and/or Cannonau grape following step c2)

5. The composition according to claim 4, **characterised in that** step c1) is performed by a microwave treatment, advantageously microwaves with a high effective power of between 1 kWatt and 5 kWatts per kg of mixture to be treated are used.

6. The composition according to any of claims 4 to 5, **characterised in that** step c2) comprises an ultrasonic cavitation treatment; in particular, the cavitation frequency is less than 50 kHz, preferably between 20 kHz and 30 kHz.

7. The composition according to any of the preceding claims, **characterised in that** the grape hydrophilic concentrate is obtained by a method comprising the following successive steps:
i. Preparing a juice of Tannat and/or Cannonau grape berries;
ii. Heating to a temperature of between 50°C and 200°C the juice obtained following step i.;
iii. Recovering the hydrophilic concentrate
iv. Advantageously, adding a preservative such as chlorphenesin to the concentrate.

8. The composition according to claim 7, **characterised in that** step ii) is performed by a microwave treatment, advantageously microwaves with a high effective power of between 300 Watts and 6,000 Watts per kg of mixture to be treated are used.

9. The composition according to any of the preceding claims, **characterised in that** the mass ratio (oily extract):(hydrophilic concentrate) ranges from 10:1 to 1:10.

10. The composition according to any of the preceding claims, **characterised in that** it further comprises one or more excipients selected from fat-soluble vitamin C, carnosic acid, tocopherol, free ascorbic acid, salt or ester, free citric or lactic acid or ester, natural preservatives such as chlorphenesin.

11. The composition according to any of the preceding claims for use as an antioxidant agent, advantageously to prevent oxidative stress.

12. A solvent-free method for preparing a physically and microbiologically stabilised grape hydrophilic concentrate comprising the following successive steps:
i. Preparing a juice of Tannat and/or Cannonau grape berries;
ii. Microwave heating, to a temperature of between 80°C and 130°C, the juice obtained following step i.;
iii. Recovering the hydrophilic concentrate,
iv. Adding a preservative, such as chlorphenesin, to the concentrate.

13. A hydrophilic concentrate of grape berries, of Tannat or Cannonau variety, obtained by the method according to claim 12, comprising at least 0.5 g/100 g of OPC expressed in eq. Procyanidin.
